# EUROPEAN PATENT SPECIFICATION

(11) **EP 2 062 866 B1**
(45) Date of publication and mention of the grant of the patent: **28.03.2012**
(21) Application number: 08168778.2
(22) Date of filing: 10.11.2008
(51) Int. Cl.: C07C 17/087, C07C 17/21, C07C 17/25, C07C 21/18, C07C 19/08

(54) **Gas phase synthesis of 2,3,3,3-tetrafluoro-1-propene from 2-chloro-3,3,3-trifluoro-1-propene**
Gasphasensynthese von 2,3,3,3-Tetrafluor-1-propen aus 2-Chlor-3,3,3-trifluor-1-propen
Synthèse en phase gazeuse de 2,3,3,3-tétrafluoro-1-propène à partir de 2-chloro-3,3,3-trifluoro-1-propène

(30) Priority: 09.11.2007 US 986599 P; 05.11.2008 US 265335
(43) Date of publication of application: 27.05.2009
(73) Proprietor: Honeywell International Inc., Morristown, NJ 07962 (US)
(72) Inventor: Mukhopadhyay, Sudip, Berkeley, CA 94702 (US); Light, Barbara A, Niagara Falls, NY 14305 (US); Fleming, Kim M, Hamburg, NY 14075 (US); Phillips, Steven D, Buffalo, NY 14218 (US); Dubey, Rajesh K, Williamsville, NY 14221 (US)
(74) Representative: Hucker, Charlotte Jane

(56) References cited:
- WO-A-2007/079431

## Description

### BACKGROUND

### Field of Invention:

This invention relates to methods of synthesizing hydrofluoroolefins. More particularly, the invention relates to catalytic synthesis of 2,3,3,3-tetrafluoro-1-propene.

### Description of Related Art:

The hydrofluoroolefin 2,3,3,3-tetrafluoro-1-propene (HFO-1234yf), which can be used as a low global warming potential (GWP) refrigerant, blowing agent, etc., has been synthesized by the catalytic pyrolysis of methyl chloride and either tetrafluoroethylene or chlorodifluoromethane. For example, US 2,931,840 describes concurrently passing 55 cc/min of MeCl and 110 cc/min CHClF₂ through a platinum tube (6 mm x 24 in) that is heated to about 800°C. The gaseous product synthesized by this process is scrubbed to remove HCl and dried, but yields only a small amount (14.8 mole %) of 2,3,3,3-tetrafluoropropene. This low yield correlates to almost 90% of the starting material being lost to unimportant byproducts including a significant amount of carbon black, which tends to deactivate the reaction's catalyst.

EP 328148 discloses that fluorine-containing olefins having the formula CH₂=CFR_{f} [R_{f} = (per) halo alkyl group)] can be prepared at low cost and with low toxic waste generation by high-temperature dehydrohalogenation and dehydration of fluoroalcohols such as HOCH₂CF₂R_{f} in the presence of H₂ gas.

JP 06072925 describes the synthesis of 2,3,3,3-tetrafluoro-1-propanol by reacting tetrafluoroethylene, formaldehyde, and HF in the presence of a TiF₄ catalyst and limonene as a polymer inhibitor. The 2,3,3,3-tetrafluoro-1-propanol, in turn, can serve as a starting material to synthesize 2,3,3,3-tetrafluoro-1-propene (HFO-1234yf). The process of JP 06072925 involves feeding a 1:3 (mol/mol) gaseous mixture of HOCH₂(CF₂)₄H and H₂ into a tube reactor packed with activated carbon and heated to 500 °C. A residence time of 4 seconds yields CH₂=CF(CF₂)₃H at a monomer conversion of 64 % and a selectivity of 82 %. Although this is a high-yield process, commercial handling of H₂ at high temperature is exceptionally hazardous. In addition, the process of JP 06072925 requires a costly on-site generation of H₂.

WO 2007/079431 A2 discloses processes for the production of fluorinated olefins, preferably adapted to commercialization of CF₃CF=CH₂ (1234yf). Three steps may be used in preferred embodiments in which a feedstock such as CCl₂=CClCH₂Cl (which may be purchased or synthesized from 1,2,3- trichloropropane) is fluorinated (preferably with HF in gas-phase in the presence of a catalyst) to synthesize a compound such as CFaCCl=CH₂, preferably in a 80.96% selectivity. The CF₃CCl=CH₂ is preferably converted to CF₃CFClCH₃ (244-isomer) using a SbCl₅ as the catalyst which is then transformed selectively to 1234yf, preferably in a gas-phase catalytic reaction using activated carbon as the catalyst. For the first step, a mixture of Cr₂O₃ and FeCl₃C is preferably used as the catalyst to achieve high selectivity to CF₃CCl=CH₂ (96%). In the second step, SbCl5/C is preferably used as the selective catalyst for transforming 1233xf to 244-isomer, CF₃CFClCH₃. The intermediates are preferably isolated and purified by distillation and used in the next step without further purification, preferably to a purity level of greater than about 95%.

Therefore, there is a need for alternative commercial processes for producing HFO-1234yf that involving more economical and less hazardous starting materials which also result in higher conversion rates and selectivity.

### SUMMARY OF THE INVENTION

Applicants have found a commercially viable process for manufacturing HFC-1234yf from HFO-1233xd that results in a conversion of over 90 % and a selectivity of up to about 80 %. More particularly, Applicants have found a two-step continuous gas-phase process for the production of HFO-1234yf wherein (1) relatively inexpensive HFO-1233xd is reacted with HF in the presence of a first catalyst to form an intermediate composition comprising, for example, CF₃CFClCH₃ and/or CF₃CF₂CH₃, at least a portion of unreacted HF is removed from the intermediate composition and (2) in the presence of a second catalyst, transforming the intermediate composition into a product comprising the desired HFO-1234yf.

Accordingly, an aspect of the invention involves a process for preparing 2,3,3,3-tetrafluoro-1-propene comprising: (a) contacting a starting material comprising 2-chloro-3,3,3-trifluoro-1-propene with hydrogen fluoride in the presence of a first activated catalyst selected from the group consisting of antimony-halides, iron-halides, titanium halides, and tin-halides, to produce an intermediate composition, removing at least a portion of unreacted HF from said intermediate composition; and (b) contacting at least a portion of the intermediate composition with a second activated catalyst comprising carbon to produce a final product comprising 2,3,3,3-tetrafluoro-1-propene.

### BRIEF DESCRIPTION OF THE DRAWINGS

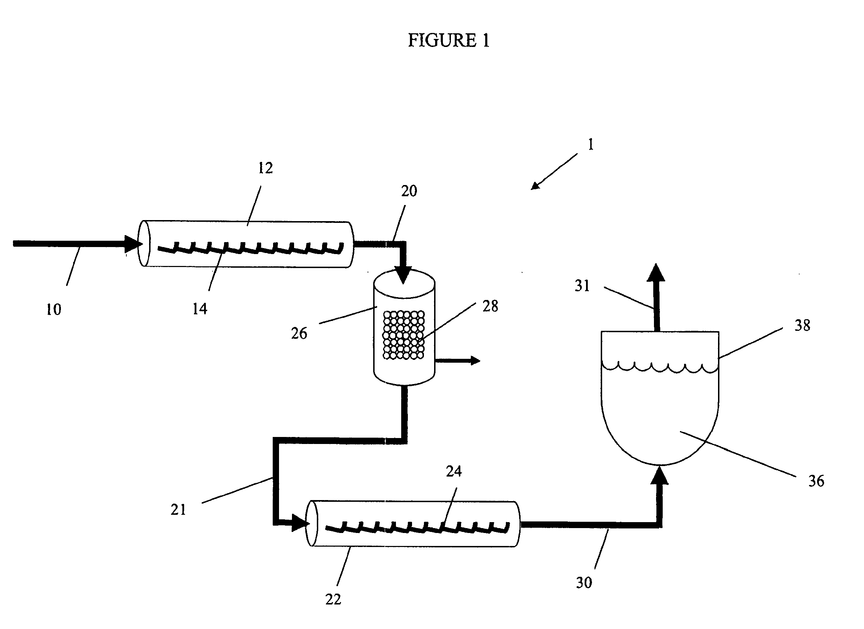

FIG. 1 shows a schematic diagram of a preferred 2,3,3,3-tetrafluoro-1-propene synthesis process according to the invention.

### DETAILED DESCRIPTION OF PREFERRED EMBODIMENTS OF THE INVENTION

Provided is a multi-step process for preparing 2,3,3,3-tetrafluoro-1-propene comprising two catalytic reactions. Preferably, the two catalytic reactions of the process are conducted separate reactors that are arranged in series. Referring to Figure 1, the shown preferred process for 2,3,3,3-tetrafluoro-1-propene synthesis 1 involves one or more feed stream(s) 10 of HFC-1233xd and HF that are introduced into a first reactor 12, having a first catalyst bed 14, to produce an intermediate composition steam 20 comprising, for example, CF₃CFClCH₃ and/or CF₃CF₂CH₃. This intermediate composition stream 20 exits from the first reactor 12 and is then passed through column 26 which is packed with an HF scavenger 26, such as NaF, KF, or Al₂O₃, to remove a majority of, and preferably substantially all, the unreacted HF exiting the first reactor 12. The resulting cleaned intermediate composition stream 21 is then fed into a second reactor 22 having a second catalyst bed 24 to produce a final product stream 30 comprising HFO-1234yf. The final product steam 30 recovered from the second reactor 22 may optionally be passed through a scrubber 38 containing, for example, a KOH solution 36, to scrub HF and/or HCl from the steam, thereby yielding a cleaned final product stream 31.

Preferred catalysts for the first reaction include, but are not limited to, antimony halides, iron halides, titanium halides, and tin halides. More preferred catalysts are SbCl₅, FeCl₃, TiCl₄, and SnCl₄. Preferred antimony halides include antimony pentahalides, particularly those having the formula: SbX₅, wherein X is independently selected from Cl and F, with SbCl₅ being most preferred. Preferably, the first catalyst is supported by a substrate, such as carbon. Also, preferably the catalyst is arranged as bed that is disposed within the first reactor.

In highly preferred embodiments, the first catalyst is pretreated (i.e., activated) with Cl₂ and/or HF, and more preferably with both Cl₂ and HF, prior to use in the synthesis reaction. For example, an SbCl₅ catalyst supported on a carbon substrate can be activated by exposing the catalyst to 50 g/h of HF at 65 °C for 4 hours, then with the combination of about 50 g/h of HF and about 200 seem of Cl₂ at about 65 °C for about 4 hours.

After pretreatment, free chloride is preferably removed from the catalyst surface. For example, after pretreatment about 50 seem of N₂ can be passed over the catalyst bed for about 40 minutes to remove substantially all of the free chloride.

After the intermediate composition stream exits the first reactor, it is processed to remove residual and/or unreacted HF from the stream. For example the intermediate composition stream can be passed through a column packed with NaF, KF, or Al₂O₃. The temperature of the packed column is preferably maintained at about 50 - 75 °C for high HF absorption efficiency.

Preferred catalysts for the second reaction are activated carbons. Acceptable activated carbons include those produced by Takeda Chemical Industries (marketed under the trade name SHIRO SAGI), Calgon Carbon Corp. (marketed under the trade name CARBOSORB^{™}), Norit Americas Inc., and Aldrich (marketed under the trade name DARCO^{®}). The second catalysts is preferably arranged as a bed that is disposed within the second reactor.

In one embodiment, the flow of material through the reactors is controlled by regulating the flow of the exit gases from the second reactor via a control valve, thus establishing a pressure drop though the system. That is, the flow of materials through the system is controlled by limiting the amount of material exiting the last second reactor and, thus, controlled pressure drops between subsequent components in the system is achieved. Preferably, the pressure in the first reactor is maintained at between about 2.76 bar (40) and about 6.89 bar (100 psig), more preferably from about 2.76 bar (40) to 3.45 bar (50 psig), whereas the pressure in the second reactor is maintained at between about 0 and about 6.89 bar (100 psig), more preferably from about 0.35 (5) to 0.69 bar (10 psig), provided that the pressure in the second reactor is lower than the pressure in the first reactor.

Preferably, the temperature of the first reaction is maintained at about 140 to 200 °C, more preferably from about 150 to 185° C. The temperature of the second reactor is preferably maintained at about 400 to 600 °C, more preferably from about 450 to 550 °C.

### EXAMPLES

Certain aspects of the present invention are further illustrated, but are not limited by, the following examples.

### Example 1:

A 22-inch (½-inch diameter) Monel tube reactor, serving as the first reactor, was charged with about 120 cc of a 50-wt% SbCl₅/Ccatalyst. The reactor was mounted inside a heater to maintain the reactor temperature. The temperature of the reaction was monitored by a thermocouple disposed at the middle inside of the reactor. The inlet of the reactor was connected to an electrical preheater, which maintained the inlet at 300 °C. The pressure inside the reactor was maintained at about 3.10 bar (45 psig).

A stream of HFO-1233xf at 70 °C and 150 seem was fed from a cylinder through a regulator, needle valve, and a gas mass-flow-meter, and into the first reactor. This feed stream kept at a constant temperature of 73°C by electrical heating to avoid condensation.

A stream of liquid HF was fed from a cylinder pressurized with N₂ at a constant pressure of 3.10 bar (45 psig). The liquid HF flowed through a dip tube, needle valve, liquid mass flow meter, a research control valve, and a gate valve and then into the reactor at a rate of 50 g/h.

All feed cylinders were mounted on scales to monitor their weight by difference.

Samples of the gas mixtures exiting the first reactor were analyzed by on-line GC and GC/MS to determine their composition. It was found that the intermediate composition contained mainly CF₃CF₂CH₃ and CF₃CFClCH₃. The conversion of HFO-1233xf was about 50 to about 100% and the combined selectivity for CF₃CF₂CH₃ and CF₃CFClCH₃ was 90-97% depending on the temperature which was varied from about 148 to 175 °C (see Table A).

The product mixtures coming out of the first reactor were passed through a packed column containing NaF to separate HF from the stream. The packed column was kept at 50-75°C for high HF adsorption efficiency.

The HF-free gas stream, thus obtained, was then passed into a second 22-inch (½-inch diameter) Monel tube reactor containing 120cc of Calgon Activated Carbon. The interior temperature of the reactor was maintained at different temperatures from 400 to 550 °C (see Table A). The pressure of the second reactor was maintained at about 0.41 bar (6 psig).

The exit gases from the second reactor were then passed through a 20-60-wt% aq. KOH scrubber solution to trap HF or HCl. The exit gases from the scrubber were condensed into a cylinder that was chilled with dry ice. The products were then isolated by distillation.

The results of the these tests are provided in Table A

**TABLE A**

| | **(Reactor 1)** | | **(Reactor 2)** | | **Conversion HFO-1233xf** | **Selectivity HFO-1234yf** |
|---|---|---|---|---|---|---|
| | **Catalyst** | **Temp.** | **Catalyst** | **Temp.** | | |
| | | (°C) | | (°C) | (%) | (%) |
| 1 | 50 wt% SbCl₅/C | 152 | Calgon | 400 | 83 | 55 |
| 2 | 50 wt% SbCl₅/C | 155 | Calgon | 450 | 86 | 57 |
| 3 | 50 wt% SbCl₅/C | 153 | Calgon | 500 | 89 | 59 |
| 4 | 50 wt% SbCl₅/C | 148 | Calgon | 500 | 88 | 60 |
| 5 | 50 wt% SbCl₅/C | 156 | Calgon | 550 | 90 | 77 |
| 6 | 50 wt% SbCl₅/C | 175 | Calgon | 550 | 93 | 79 |
| | | | | | | |

As seen in the data presented in Table A, the process described herein can be used to produce HFO-1234yf from HFO-1233xf at a conversion of over 90 % and a selectivity of about 80%.

The separation of HF from the gas stream coming out off the first reactor is highly preferred for achieving high selectivity. Only 49% selectivity for HFO-1234yfwas obtained when the exiting gas-stream of the first reactor was sent directly into the second reactor compare with 79 % selectivity when HF separation was utilized.

## Claims

1. A process for preparing 2,3,3,3-tetrafluoro-1-propene comprising:
fluorinating a starting material comprising 2-chloro-3,3,3-trifluoro-1-propene by contacting said starting material with hydrogen fluoride in the presence of a first activated catalyst selected from the group consisting of antimony-halides, iron-halides, titanium halides, and tin-halides, to produce an intermediate composition;
removing at least a portion of unreacted HF from said intermediate composition; and
dehydrohalogenating at least a portion of said intermediate composition by contacting said intermediate composition with a second activated catalyst comprising carbon to produce a final product comprising 2,3,3,3-tetrafluoro-1-propene; wherein the two-step process is a continuous gas-phase process.

2. The process of claim 1 wherein said intermediate composition comprises at least one of CF₃CFClCH₃ and CF₃CF₂CH₃.

3. The process of claim 1 wherein said intermediate composition comprises both CF₃CFClCH₃ and CF₃CF₂CH₃.

4. The process of claim 1 wherein said first catalyst is supported on carbon.

5. The process of claim 1 wherein said first catalyst has the formula: SbX₅, wherein X is independently selected from Cl and F.

6. The process of claim 5 wherein said first catalyst is SbCl₅.

7. The process of claim 1 wherein removing at least a portion of unreacted HF from said intermediate composition involves contacting said intermediate composition with at least one of NaF, KF, or Al₂O₃ at a temperature of about 20 to 100 °C.

8. The process of claim 1 wherein said process has a conversion of said 2-chloro-3,3,3-trifluoro-1-propene of at least about 80% and a selectivity for 2,3,3,3-tetrafluoropropene of at least about 55%.

## Patentansprüche

1. Verfahren zur Herstellung von 2,3,3,3-Tetrafluor-1-propen, bei dem man:
einen Ausgangsstoff, der 2-Chlor-3,3,3-trifluor-1-propen umfasst, fluoriert, indem man den Ausgangsstoff in Gegenwart eines ersten aktivierten Katalysators aus der Gruppe bestehend aus Antimonhalogeniden, Eisenhalogeniden, Titanhalogeniden und Zinnhalogeniden mit Fluorwasserstoff in Berührung bringt, wobei man eine Zwischenzusammensetzung erhält;
mindestens einen Teil des nicht umgesetzten HF aus der Zwischenzusammensetzung entfernt und
mindestens einen Teil der Zwischenzusammensetzung dehydrohalogeniert, indem man die Zwischenzusammensetzung mit einem zweiten aktivierten Katalysator, der Kohlenstoff umfasst, in Berührung bringt, wobei man ein Endprodukt, das 2,3,3,3-Tetrafluor-1-propen umfasst, erhält; wobei es sich bei dem zweischrittigen Verfahren um ein kontinuierliches Gasphasenverfahren handelt.

2. Verfahren nach Anspruch 1, bei dem die Zwischenzusammensetzung CF₃CFClCH₃ und/oder CF₃CF₂CH₃ umfasst.

3. Verfahren nach Anspruch 1, bei dem die Zwischenzusammensetzung sowohl CF₃CFClCH₃ als auch CF₃CF₂CH₃ umfasst.

4. Verfahren nach Anspruch 1, bei dem der erste Katalysator auf Kohlenstoff geträgert ist.

5. Verfahren nach Anspruch 1, bei dem der erste Katalysator die Formel SbX₅, worin X unabhängig unter Cl und F ausgewählt ist, aufweist.

6. Verfahren nach Anspruch 5, bei dem es sich bei dem ersten Katalysator um SbCl₅ handelt.

7. Verfahren nach Anspruch 1, bei dem man bei der Abtrennung von mindestens einem Teil des nicht umgesetzten HF von der Zwischenzusammensetzung die Zwischenzusammensetzung bei einer Temperatur von etwa 20 bis 100°C mit NaF, KF und/oder Al₂O₃ in Berührung bringt.

8. Verfahren nach Anspruch 1, bei dem das Verfahren einen 2-Chlor-3,3,3-trifluor-1-propen-Umsatz von mindestens etwa 80% und eine 2,3,3,3-Tetrafluorpropen-Selektivität von mindestens etwa 55% aufweist.

## Revendications

1. Procédé de préparation de 2,3,3,3-tétrafluoro-1-propène comprenant :
la fluoration d'un produit de départ comprenant du 2-chloro-3,3,3-trifluoro-1-propène en mettant en contact ledit produit de départ avec du fluorure d'hydrogène en présence d'un premier catalyseur activé choisi dans le groupe constitué par les halogénures d'antimoine, les halogénures de fer, les halogénures de titane et les halogénures d'étain, pour produire une composition intermédiaire ;
l'élimination d'au moins une partie du HF n'ayant pas réagi de ladite composition intermédiaire ; et
la déshydrohalogénation d'au moins une partie de ladite composition intermédiaire en mettant en contact ladite composition intermédiaire avec un deuxième catalyseur activé comprenant du carbone pour produire un produit final comprenant du 2,3,3,3-tétrafluoro-1-propène ; lequel procédé en deux étapes est un procédé en phase gazeuse continu.

2. Procédé selon la revendication 1 dans lequel ladite composition intermédiaire comprend au moins un des composés CF₃CFClCH₃ et CF₃CF₂CH₃.

3. Procédé selon la revendication 1 dans lequel ladite composition intermédiaire comprend les deux composés CF₃CFClCH₃ et CF₃CF₂CH₃.

4. Procédé selon la revendication 1 dans lequel ledit premier catalyseur est supporté sur du carbone.

5. Procédé selon la revendication 1 dans lequel ledit premier catalyseur a pour formule : SbX₅, dans laquelle X est choisi indépendamment parmi Cl et F.

6. Procédé selon la revendication 5 dans lequel ledit premier catalyseur est SbCl₅.

7. Procédé selon la revendication 1 dans lequel l'élimination d'au moins une partie de HF n'ayant pas réagi de ladite composition intermédiaire consiste à mettre en contact ladite composition intermédiaire avec au moins un des composés NaF, KF ou Al₂O₃ à une température d'environ 20 à 100 °C.

8. Procédé selon la revendication 1 dans lequel ledit procédé présente un taux de conversion dudit 2-chloro-3,3,3-trifluoro-1-propène d'au moins environ 80% et une sélectivité pour le 2,3,3,3-tétrafluoropropène d'au moins environ 55%.
